# EUROPÄISCHE PATENTSCHRIFT

(11) **EP 1 212 031 B1**
(45) Veröffentlichungstag und Bekanntmachung des Hinweises auf die Patenterteilung: **04.08.2004**
(21) Anmeldenummer: 00964141.6
(22) Anmeldetag: 08.09.2000
(51) Int. Cl.: A61K 7/13

(54) **VERWENDUNG VON 2-NITRO-P-PHENYLENDIAMINDERIVATEN ALS DIREKTZIEHENDE FARBSTOFFE**
USE OF 2-NITRO-P-PHENYLENE DIAMINE DERIVATIVES AS DIRECT COLORANTS
UTILISATION DE DERIVES DE 2-NITRO-P-PHENYLENEDIAMINE EN TANT QUE COLORANTS DIRECTS

(30) Priorität: 17.09.1999 DE 19944527
(43) Veröffentlichungstag der Anmeldung: 12.06.2002
(73) Patentinhaber: Henkel Kommanditgesellschaft auf Aktien, 40589 Düsseldorf-Holthausen (DE)
(72) Erfinder: NAUMANN, Frank, 40593 Düsseldorf (DE); ROSE, David, 40723 Hilden (DE); MEINIGKE, Bernd, 51381 Leverkusen (DE); HÖFFKES, Horst, 40595 Düsseldorf (DE)
(86) Internationale Anmeldenummer: PCT/EP2000/008773
(87) Internationale Veröffentlichungsnummer: WO 2001/021143

(56) Entgegenhaltungen:
- DE-A- 19 804 085
- GB-A- 1 349 118

## Beschreibung

Die Erfindung betrifft die Verwendung von 1-(N-cyclopentylamino)-2-nitro-4-aminobenzol in Haarfärbe- und Haartönungsmitteln zur Verschiebung der Nuancen in den Rotbereich sowie entsprechende Mittel.

Zubereitungen zum Tönen und Färben von Haaren sind ein wichtiger Typ von kosmetischen Mitteln. Sie können dazu dienen, die natürliche Haarfarbe gemäß den Wünschen der entsprechenden Person leicht oder stärker zu nuancieren, eine gänzlich andere Haarfarbe zu erzielen oder unerwünschte Farbtöne, wie beispielsweise Grautöne, zu überdecken. Übliche Haarfärbemittel werden, je nach gewünschter Farbe bzw. Dauerhaftigkeit der Färbung, entweder auf Basis von Oxidationsfarbstoffen oder auf Basis von direktziehenden Farbstoffen formuliert. Häufig werden auch Kombinationen von Oxidationsfarbstoffen und direktziehenden Farbstoffen zur Erzielung spezieller Nuancen eingesetzt.

Gute Farbstoffe zeichnen sich durch hohe Farbstärke aus. Weiterhin sind gute Schweiß-, Wasch- und Lichtechtheit gewünscht. Ferner sollten sie in toxikologischer und dermatologischer Einsicht unbedenklich sein. Es ist auch von Vorteil, wenn die Substanzen eine hohe Löslichkeit in verschiedenen Basisformulierungen besitzen.

Färbemittel auf Basis von Oxidationsfarbstoffen führen zu brillanten und dauerhaften Farbtönen. Sie bedingen allerdings den Einsatz starker Oxidationsmittel, wie beispielsweise Wasserstoffperoxid-Lösungen. Dies kann das zu färbende Haar schädigen. Diesen Schädigungen muß dann mit entsprechenden Pflegeprodukten entgegengewirkt werden. Außerdem können Kontakte der Haut mit diesen Färbemitteln bei sehr empfindlichen Personen zu unerwünschten Reaktionen führen.

Färbemittel auf der Basis direktziehender Farbstoffe kommen ohne Oxidationsmittel aus und können bei pH-Werten im Bereich des Neutralpunktes formuliert werden. Ein wesentlicher Nachteil der Färbemittel auf Basis direktziehender Farbstoffe ist die geringe Waschechtheit der erzielten Färbungen. Das Aufziehvermögen der Farbstoffmoleküle auf das Haar sowie der Glanz der gefärbten Haare können in vielen Fällen ebenfalls nicht voll befriedigen.

Als direktziehende Farbstoffe werden häufig 2-Nitro-p-phenylendiaminderivate verwendet. Diese Derivate sind jedoch häufig in Wasser nur unzureichend löslich oder dispergierbar. Wenn der Farbstoff im Färbemedium nicht solubilisierbar ist, führt dies zu ungleichmäßigen Färbungen und es besteht ein hohes Risiko, nur schwache Ausfärbungen zu erreichen.

Zur Verbesserung der Egalisierung sowie zur Erweiterung des Nuancenspektrums werden direktziehende Farbstoffe häufig auch in Oxidationshaarfarben eingesetzt. Allerdings weisen die üblicherweise eingesetzten direktziehenden Farbstoffe nur eine geringe Stabilität gegen Reduktions- und Oxidationsmittel auf.

Zur Erzielung modischer Tönungen ist es notwendig, rote Farbtöne zu erzielen. Daher werden stets neue direktziehende Farbstoffe gesucht, die die oben beschriebenen Nachteile nicht aufweisen und gleichzeitig intensive Ausfärbungen im Rotbereich ergeben.

GB-A-1 349 118 beschreibt die Verwendung von 1 (N-hydroxyethylamino)-2-nitro-4-aminobenzolen in Haarfärbe- und Haartönungsmitteln zur Verschiebung der Nuancen in den Rotbereich sowie entsprechende Mittel.

Es wurde nun überraschenderweise gefunden, daß bestimmte 2-Nitro-p-phenylendiamin-Derivate die an direktziehende Farbstoffe gestellten Anforderungen in einem hohen Maße erfüllen.

Ein erster Gegenstand der vorliegenden Erfindung ist daher die Verwendung von 2-Nitro-p-Phenylendiaminderivaten der Formel (I), 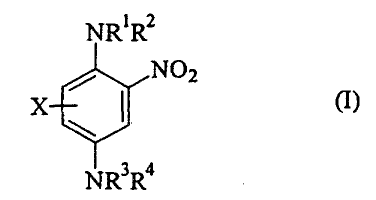 in der R¹ bis R⁴ voneinander unabhängig stehen für Wasserstoff, eine C₁₋₄-Hydroxyalkylgruppe oder einen gesättigten, einfach oder mehrfach ungesättigten C₅-Ring, der gege-benenfalls mit einer C₁₋₄-Alkylgruppe, einem Halogenatom, einer Hydroxygruppe und/oder einer Aminogruppe substituiert sein kann, und X steht für Wasserstoff oder ein Halogenatom, mit der Maßgabe, daß mindestens einer der Substituenten R¹ bis R⁴ ein C₅-Ring ist, als direktziehender Farbstoff zur Verschiebung von Haarfärbungen und - tönungen in den Rotbereich.

Unter einer Verschiebung der Haarfärbungen und -tönungen in den Rotbereich wird erfindungsgemäß verstanden, daß die Verbindungen der Formel (I) einen rötlichen Beitrag zur Gesamtfärbung leisten. Dies äußert sich durch einen stärker positiven a-Wert der Färbung im CIELAB-Farbraum. Erfindungsgemäß kann dies auch verwirklicht sein, wenn die Gesamtfärbung selber kein Rot/Braunton, sondern beispielsweise eine schwarze Nuance ist.

Beispiele für die als Substituenten in den erfindungsgemäßen Verbindungen genannten, C₁- bis C₄-Alkydgruppen sind die Gruppen Methyl, Ethyl, Propyl, Isopropyl und Butyl. Ethyl und Methyl sind bevorzugte Alkylgruppen. Eine Methylgruppe ist besonders bevorzugt. Als bevorzugte Beispiele für eine C₁- bis C₄-Hydroxyalkylgruppe können eine Hydroxymethyl-, eine 2-Hydroxyethyl-, eine 3-Hydroxypropyl- oder eine 4-Hydroxybutylgruppe genannt werden, eine 2-Hydroxyethylgruppe ist besonders bevorzugt. Beispiele für ein Halogenatom sind erfindungsgemäß ein F-, ein Cl- oder ein Br-Atom, ein Cl-Atom ist besonders bevorzugt. Bevorzugte C₅-Ringe sind der Cyclopentylring sowie dessen methyl-, hydroxy- oder amino-substituierten Derivate. Der unsubstituierte Cyclopentylring ist besonders bevorzugt.

Besonders bevorzugt sind Verbindungen der Formel (I), bei denen die Reste R² bis R⁴ für Wasserstoff stehen. Dennoch sind auch Verbindungen der Formel (I), bei denen R¹ und R³ jeweils für einen Ringsubstituenten stehen, und Verbindungen, bei denen einer der Reste R¹ oder R³ für ein Ringsystem und der andere der Reste R¹ oder R³ für eine 2-Hydroxyethylgruppe stehen, bevorzugt.

Bevorzugt sind ferner Verbindungen der Formel (I), die einen Chlorsubstituenten (X) an der Position 3, 5 oder 6 tragen.
Besonders bevorzugt sind Verbindungen der Formel (I), bei denen X für ein Wasserstoffatom steht.

Eine ganz besonders bevorzugte Verbindung der Formel (I) ist 1-(N-cyclopentylamino)-2-nitro-4-aminobenzol.

Verbindungen der Formel (I) zum Färben und Tönen keratinischer Fasern sind bereits aus der GB-1 206 491 bekannt. Dieser sehr allgemeinen Schrift sind aber keinerlei Hinweise auf die hervorragenden färberischen Eigenschaften, insbesondere auf die brillanten roten Färbungen, der hier beanspruchten C₅-Ringverbindungen zu entnehmen.

Ein zweiter Gegenstand sind ferner Mittel zum Färben keratinischer Fasern, insbesondere menschlicher Haare, die als direktziehenden Farbstoff mindestens ein 2-Nitro-p-Phenylendiaminderivat der Formel (I), 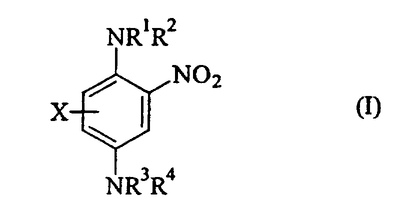 in der R¹ bis R⁴ voneinander unabhängig stehen für Wasserstoff, eine C₁₋₄-Hydroxyalkylgruppe oder einen gesättigten, einfach oder mehrfach ungesättigten C₅-Ring, der gegebenenfalls mit einer C₁₋₄-Alkylgruppe, einem Halogenatom, einer Hydroxygruppe und/oder einer Aminogruppe substituiert sein kann, und X steht für Wasserstoff oder ein Halogenatom, mit der Maßgabe, daß mindestens einer der Substituenten R¹ bis R⁴ ein C₅-Ring ist, enthalten.

Zur Erläuterung der bevorzugten Verbindungen der Formel (I) sei an dieser Stelle explizit auf die obigen Ausführungen verwiesen.

Gemäß einer ersten bevorzugten Ausführungsform dieses Gegenstandes der Erfindung handelt es sich um solche Mittel, die nur eine vorübergehende Färbung der Faser bewirken sollen. Solche Mittel werden häufig als Tönungsmittel bezeichnet. Diese Ausführungsform umfaßt beispielsweise auch solche Haarbehandlungsmittel, mit denen die Haare nicht nur vorübergehend gefärbt, sondern auch zu einer bestimmten Frisur gestylt werden sollen. In diesem Falle spricht man von Tönungsfestigern.

Da solche Mittel üblicherweise ohne die Zuhilfenahme von oxidierenden Komponenten, insbesondere Wasserstoffperoxid, formuliert werden, können die erfindungsgemäßen Mittel gemäß dieser Ausführungsform frei von Oxidationsfarbstoffvorprodukten sein.

Wenngleich die Verbindungen gemäß Formel (I) auch als alleinige Farbstoffkomponente eingesetzt werden können, so enthalten die Mittel gemäß dieser Ausführungsform bevorzugt noch mindestens einen weiteren Farbstoff vom Typ der Direktzieher.

Direktziehende Farbstoffe sind üblicherweise Nitrophenylendiamine, Nitroaminophenole, Azofarbstoffe, Anthrachinone oder Indophenole. Bevorzugte direktziehende Farbstoffe sind die unter den internationalen Bezeichnungen bzw. Handelsnamen HC Yellow 2, HC Yellow 4, HC Yellow 5, HC Yellow 6, Basic Yellow 57, Disperse Orange 3, HC Red 3, HC Red BN, Basic Red 76, HC Blue 2, HC Blue 12, Disperse Blue 3, Basic Blue 99, HC Violet 1, Disperse Violet 1, Disperse Violet 4, Disperse Black 9, Basic Brown 16 und Basic Brown 17 bekannten Verbindungen sowie 1,4-Bis-(β-hydroxyethyl)-amino-2-nitrobenzol, 4-Amino-2-nitrodiphenylamin-2'-carbonsäure, 6-Nitro-1,2,3,4-tetrahydrochinoxalin, Hydroxyethyl-2-nitro-toluidin, Pikraminsäure, 2-Amino-6-chloro-4-nitrophenol, 4-Ethylamino-3-nitrobenzoesäure und 2-Chloro-6-ethylamino-1-hydroxy-4-nitrobenzol. Die erfindungsgemäßen Mittel gemäß dieser Ausführungsform enthalten die direktziehenden Farbstoffe, bevorzugt in einer Menge von 0,01 bis 20 Gew.-%, bezogen auf das gesamte Färbemittel.

Weiterhin können die erfindungsgemäßen Zubereitungen auch in der Natur vorkommende Farbstoffe, wie beispielsweise Henna rot, Henna neutral, Henna schwarz, Kamillenblüte, Sandelholz, schwarzen Tee, Faulbaumrinde, Salbei, Blauholz, Krappwurzel, Catechu, Sedre und Alkannawurzel enthalten.

Weitere in den erfindungsgemäßen Färbemitteln enthaltene Farbstoffkomponenten können auch Indole und Indoline, sowie deren physiologisch verträgliche Salze, sein. Bevorzugte Beispiele sind 5,6-Dihydroxyindol, N-Methyl-5,6-dihydroxyindol, N-Ethyl-5,6-dihydroxyindol, N-Propyl-5,6-dihydroxyindol, N-Butyl-5,6-dihydroxyindol. 5,6-Dihydroxyindol-2-carbonsäure, 6-Hydroxyindol, 6-Aminoindol und 4-Aminoindol. Weiterhin bevorzugt sind 5,6-Dihydroxyindolin, N-Methyl-5,6-dihydroxyindolin, N-Ethyl-5.6-dihydroxyindolin, N-Propyl-5,6-dihydroxyindolin, N-Butyl-5,6-dihydroxyindolin, 5,6-Dihydroxyindolin-2-carbonsäure, 6-Hydroxyindolin, 6-Aminoindolin und 4-Aminoindolin.

Gemäß einer zweiten bevorzugten Ausführungsform handelt es sich bei den beanspruchten Mitteln um Haarfärbemittel für die dauerhafte Färbung der Haare. Diese Mittel enthalten mindestens ein Oxidationsfarbstoffvorprodukt vom Typ der Entwickler. Diese, in der Regel farblosen, Verbindungen reagieren unter der Einwirkung von Oxidationsmitteln oder von Luftsauerstoff, gegebenenfalls mit Hilfe spezieller Enzyme oder Metallionen als Katalysator, mit sich unter der Ausbildung der gewünschten Farbstoffe. Insbesondere zur Ausbildung natürlicher Haarfarbtöne werden aber in der Regel Kombinationen von mehreren Entwicklerkomponenten eingesetzt. Weiterhin werden in der Regel zusätzlich sogenannte Kuppler eingesetzt, die unter dem Einfluß von Oxidationsmitteln mit den Entwicklerkomponenten reagieren, was zu neuen Farben bzw. einer Nuancierung der Farbe führt. Erfindungsgemäß kann sowohl eine Kupplerkomponente als auch mehrere Kupplerkomponenten in Kombination mit einer oder mehreren Entwicklerkomponenten eingesetzt werden.

Erfindungsgemäß bevorzugte Entwicklerkomponenten sind p-Phenylendiamin, p-Toluylendiamin, p-Aminophenol, o-Aminophenol, 1-(2'-Hydroxyethyl)-2,5-diaminobenzol, N,N-Bis-(2-hydroxy-ethyl)-p-phenylendiamin, 2-(2,5-Diaminophenoxy)-ethanol, 1-Phenyl-3-carboxyamido-4-amino-pyrazolon-5, 4-Amino-3-methylphenol, 2,4,5,6-Tetraaminopyrimidin, 2-Hydroxy-4,5,6-triaminopyrimidin, 4-Hydroxy-2,5,6-triaminopyrimidin, 2,4-Dihydroxy-5,6-diaminopyrimidin, 2-Dimethylamino-4,5,6-triaminopyrimidin, 2-Hydroxymethylamino-4-amino-phenol, Bis-(4-aminophenyl)amin, 4-Amino-3-fluorphenol, 2-Aminomethyl-4-aminophenol, 2-Hydroxymethyl-4-aminophenol, 4-Amino-2-((diethylamino)-methyl)-phenol, Bis-(2-hydroxy-5-aminophenyl)-methan, 1,4-Bis-(4-aminophenyl)-diazacycloheptan, 1,3-Bis(N(2-hydroxyethyl)-N(4-aminophenylamino))-2-propanol, 4-Amino-2-(2-hydroxyethoxy)-phenol, 1,10-Bis-(2,5-diaminophenyl)-1,4,7,10-tetraoxadecan sowie 4,5-Diaminopyrazol-Derivate nach EP 0 740 931 bzw. WO 94/08970 wie z. B. 4,5-Diamino-1-(2'-hydroxyethyl)-pyrazol.

Besonders bevorzugte Entwicklerkomponenten sind erfindungsgemäß p-Phenylendiamin, p-Toluylendiamin, p-Aminophenol, 1-(2'-Hydroxyethyl)-2,5-diaminobenzol, N.N-Bis-(2-hydroxy-ethyl)-p-phenylendiamin, 4-Amino-3-methylphenol, 4-Amino-2-((diethylamino)-methyl)-phenol, 2-Aminomethyl-4-aminophenol, 2,4,5,6-Tetraaminopyrimidin, 2-Hydroxy-4,5,6-triaminopyrimidin, 4-Hydroxy-2,5,6-triaminopyrimidin und 4,5-Diamino-1-(2'-hydroxyethyl)-pyrazol.

Erfindungsgemäß bevorzugte Kupplerkomponenten sind
- m-Aminophenol und dessen Derivate wie beispielsweise 5-Amino-2-methylphenol, 3-Amino-2-chlor-6-methylphenol, 2-Hydroxy-4-aminophenoxyethanol, 2,6-Dimethyl-3-aminophenol, 3-Trifluoroacetylamino-2-chlor-6-methylphenol, 5-Amino-4-chlor-2-methylphenol, 5-Amino-4-methoxy-2-methylphenol, 5-(2'-Hydroxyethyl)-amino-2-methylphenol, 3-(Diethylamino)-phenol, N-Cyclopentyl-3-aminophenol, 1,3-Dihydroxy-5-(methylamino)-benzol, 3-(Ethylamino)-4-methylphenol und 2,4-Dichlor-3-aminophenol,
- o-Aminophenol und dessen Derivate,
- m-Diaminobenzol und dessen Derivate wie beispielsweise 2,4-Diaminophenoxyethanol, 1,3-Bis-(2,4-diaminophenoxy)-propan, 1-Methoxy-2-amino-4-(2'-hydroxyethylamino)benzol, 1,3-Bis-(2,4-diaminophenyl)-propan, 2,6-Bis-(2-hydroxyethylamino)-1-methylbenzol und 1-Amino-3-bis-(2'-hydroxyethyl)-aminobenzol,
- o-Diaminobenzol und dessen Derivate wie beispielsweise 3,4-Diaminobenzoesäure und 2,3-Diamino-1-methylbenzol,
- Di- beziehungsweise Trihydroxybenzolderivate wie beispielsweise Resorcin, Resorcinmonomethylether, 2-Methylresorcin, 5-Methylresorcin, 2,5-Dimethylresorcin, 2-Chlorresorcin, 4-Chlorresorcin, Pyrogallol und 1,2,4-Trihydroxybenzol,
- Pyridinderivate wie beispielsweise 2,6-Dihydroxypyridin, 2-Amino-3-hydroxypyridin, 2-Amino-5-chlor-3-hydroxypyridin, 3-Amino-2-methylamino-6-methoxypyridin, 2,6-Dihydroxy-3,4-dimethylpyridin, 2,6-Dihydroxy-4-methylpyridin, 2,6-Diaminopyridin, 2,3-Diamino-6-methoxypyridin und 3,5-Diamino-2,6-dimethoxypyridin,
- Naphthalinderivate wie beispielsweise 1-Naphthol, 2-Methyl-1-naphthol, 2-Hydroxymethyl-1-naphthol, 2-Hydroxyethyl-1-naphthol, 1,5-Dihydroxynaphthalin, 1,6-Dihydroxynaphthalin, 1,7-Dihydroxynaphthalin, 1,8-Dihydroxynaphthalin, 2,7-Dihydroxynaphthalin und 2,3-Dihydroxynaphthalin,
- Morpholinderivate wie beispielsweise 6-Hydroxybenzomorpholin und 6-Aminobenzomorpholin,
- Chinoxalinderivate wie beispielsweise 6-Methyl-1,2,3,4-tetrahydrochinoxalin,
- Pyrazolderivate wie beispielsweise 1-Phenyl-3-methylpyrazol-5-on,
- Indolderivate wie beispielsweise 4-Hydroxyindol, 6-Hydroxyindol und 7-Hydroxyindol,
- Methylendioxybenzolderivate wie beispielsweise 1-Hydroxy-3,4-methylendioxybenzol, 1-Amino-3,4-methylendioxybenzol und 1-(2'-Hydroxyethyl)-amino-3,4-methylendioxybenzol.

Erfindungsgemäß bevorzugte Kupplerkomponenten sind ferner 3-Dimethylaminophenol, 2,4-Dihydroxyanilin, 8-Amino-6-methoxy-chinolin, 2-Amino-5-naphthol-1,7-disulfonsäure, 3,5-Diaminobenzamid, 3-Methylsulfonylamino-2-methylanilin, 5,6-Dihydroxybenzimidazol, 2,2'-Dihydroxybenzylamin, 3,5,3',5'-Tetraamino-2,2'-dimethoxy-diphenyl, 3,5-Diamino-p-chlorbenzotrifluorid, 4-Methyl-3-aminophenol, 2,4-Diamino-3-chlorphenol, 1-Amino-3-di-(2-hydroxyethylamino)-4-ethoxybenzol, 2,4-Dimethylresorcin, Bis-(2,4-diaminophenoxy)-methan, 2,6-Bis-(hydroxyethyl)-pyridin, 4-Hydroxy-3-methoxybenzylalkohol, 8-Hydroxychinolin, 4-Hydroxy-3-methoxy-benzylamin, 4-Ethylresorcin, 2-Methylthio-5-aminophenol, 5-[(3-Hydroxypropyl)amino]-2-methylphenol, 2,6-Dimethoxy-3-aminophenol und 2,6-Diamino-3-methylthiotoluol.

Erfindungsgemäß besonders bevorzugte Kupplerkomponenten sind 1-Naphthol, 1,5-, 2,7-und 1,7-Dihydroxynaphthalin, 3-Aminophenol, 5-Amino-2-methylphenol, 2-Amino-3-hydroxypyridin, Resorcin, 4-Chlorresorcin, 2-Chlor-6-methyl-3-aminophenol, 2-Methylresorcin, 5-Methylresorcin, 2,5-Dimethylresorcin und 2,6-Dihydroxy-3,4-dimethylpyridin.

Erfindungsgemäß können solche Entwickler- und Kupplerkomponenten, die für die Ausbildung der Färbung keine Oxidationsmittel außer Luftsauerstoff benötigen, bevorzugt sein.
Die erfindungsgemäßen Mittel dieser Ausführungsform können, außer den Verbindungen gemäß Formel (I), den Entwickler und gegebenenfalls Kupplerkomponenten gewünschtenfalls zur Nuancierung noch weitere direktziehende Farbstoffe enthalten. Es sei an dieser Stelle auf die oben angegebene Auflistung verwiesen.

Die erfindungsgemäßen Haarfärbemittel enthalten sowohl die Entwicklerkomponenten als auch die Kupplerkomponenten bevorzugt in einer Menge von 0,005 bis 20 Gew.-%, jeweils bezogen auf das Färbemittel ohne die Oxidationsmittelzubereitung. Dabei werden Entwicklerkomponenten und Kupplerkomponenten im allgemeinen in etwa molaren Mengen zueinander eingesetzt. Wenn sich auch der molare Einsatz als zweckmäßig erwiesen hat, so ist ein gewisser Überschuß einzelner Oxidationsfarbstoffvorprodukte nicht nachteilig, so daß Entwicklerkomponenten und Kupplerkomponenten in einem Mol-Verhältnis von 1 : 0,5 bis 1 : 2 enthalten sein können.

Es ist nicht erforderlich, daß die Oxidationsfarbstoffvorprodukte oder die direktziehenden Farbstoffe jeweils einheitliche Verbindungen darstellen. Vielmehr können in den erfindungsgemäßen Haarfärbemitteln, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z. B. toxikologischen, ausgeschlossen werden müssen.

Bezüglich der in den erfindungsgemäßen Haarfärbe- und -tönungsmitteln einsetzbaren Farbstoffe wird weiterhin ausdrücklich auf die Monographie Ch. Zviak, The Science of Hair Care, Kapitel 7 (Seiten 248-250; direktziehende Farbstoffe) sowie Kapitel 8, Seiten 264-267; Oxidationsfarbstoffvorprodukte), erschienen als Band 7 der Reihe "Dermatology" (Hrg.: Ch., Culnan und H. Maibach), Verlag Marcel Dekker Inc., New York, Basel, 1986, sowie das "Europäische Inventar der Kosmetik-Rohstoffe", herausgegeben von der Europäischen Gemeinschaft, erhältlich in Diskettenform vom Bundesverband Deutscher Industrie- und Handelsunternehmen für Arzneimittel, Reformwaren und Körperpflegemittel e.V., Mannheim, Bezug genommen.

Gemäß einer bevorzugten Ausführungsform enthalten die erfindungsgemäßen Mittel, insbesondere, wenn sie noch konditionierende oder festigende Eigenschaften aufweisen sollten, weiterhin anionische, nichtionogene oder insbesondere kationische Polymere.

Als konditionierende Wirkstoffe geeignete kationische Polymere enthalten innerhalb des Polymergerüstes kationische Gruppen. Diese Gruppen können Teil der Polymerkette sein. sie können sich aber auch in Seitenketten befinden, die über Zwischenglieder mit einer Hauptkette verbunden sind. Übliche kationische Gruppen enthalten quartäre Stickstoff- oder Phosphoratome. Gruppen mit quartären Stickstoffatomen sind dabei bevorzugt. Die quartären Stickstoffatome können dabei sowohl 4 unterschiedliche oder z. T. gleiche Substituenten tragen, als auch Teil eines Ringsystems sein. Bevorzugte kationische Gruppen sind Ammonium- und Imidazoliumgruppen.

Beispiele für solche Polymere sind:
- quaternierte Cellulose-Derivate, wie sie unter den Bezeichnungen Celquat® und Polymer JR® im Handel erhältlich sind. Die Verbindungen Celquat® H 100, Celquat L 200 und Polymer JR®400 sind bevorzugte quaternierte Cellulose-Derivate.
- Copolymere des Vinylpyrrolidons mit quaternierten Derivaten des Dialkylaminoalkylacrylats- und -methacrylats, wie beispielsweise mit Diethylsulfat quaternierte Vinylpyrrolidon-Dimethylaminoalkylmethacrylat-Copolymere sowie das Vinylpyrrolidon-Methacrylamidopropyltrimethylammoniumchlorid-Copolymere. Solche Verbindungen sind unter den Bezeichnungen Gafquat®734, Gafquat®755 bzw. Gafquat® HS 100 im Handel erhältlich.
- Copolymerisate des Vinylpyrrolidons mit Vinylimidazoliummethochlorid, wie sie unter der Bezeichnung Luviquat® angeboten werden.
- polymere Dimethyldiallylammoniumsalze und deren Copolymere mit Acrylsäure sowie Estern und Amiden von Acrylsäure und Methacrylsäure. Die unter den Bezeichnungen Merquat®100 (Poly(dimethyldiallylammoniumchlorid)), Merquat®550 (Dimethyldiallylammoniumchlorid-Acrylamid-Copolymer) und Merquat® 280 (Dimethyldiallylammoniumchlorid-Acrylsäure-Copolymer im Handel erhältlichen Produkte sind Beispiele für solche kationischen Polymere.
- quaternierte Guar-Derivate, wie sie unter den Bezeichnungen Cosmedia Guar® und Jaguar® im Handel erhältlich sind. Bevorzugte Guar-Derivate sind beispielsweise Cosmedia Guar® C-261 und Jaguar® C 13-S.
- Kationisch derivatisierte Silikonöle, wie beispielsweise die im Handel erhältlichen Produkte Q2-7224 (Hersteller: Dow Coming; ein stabilisiertes Trimethylsilylamodimethicon), Dow Corning 929 Emulsion (enthaltend ein hydroxyl-amino-modifiziertes Silicon, das auch als Amodimethicone bezeichnet wird), SM-2059 (Hersteller: General Electric), SLM-55067 (Hersteller: Wacker) sowie Abil®-Quat 3270 und 3272 (Hersteller: Th. Goldschmidt; diquatemäre Polydimethylsiloxane, Quaternium-80).
- Chitosan und dessen Derivate
- quaternierter Polyvinylalkohol
   sowie die unter den Bezeichnungen
- Polyquatemium 2,
- Polyquaternium 17,
- Polyquaternium 18 und
- Polyquatemium 27 bekannten Polymeren mit quartären Stickstoffatomen in der Polymerhauptkette.

Besonders bevorzugt sind kationische Polymere der vier erstgenannten Gruppen, ganz besonders bevorzugt sind Polyquaternium-2, Polyquaternium-10 und Polyquaternium-22.

Bevorzugt sind die kationischen Polymeren in den erfindungsgemäßen Mitteln in Mengen von 0,1 - 5 Gew.-%, bezogen auf die gesamte Zubereitung, enthalten.

Geeignete nichtionogene Polymere sind beispielsweise:
- Vinylpyrrolidon/Vinylester-Copolymere, wie sie beispielsweise unter dem Warenzeichen Luviskol® (BASF) vertrieben werden. Luviskol® VA 64 und Luviskol® VA 73, jeweils Vinylpyrrolidon/Vinylacetat-Copolymere, sind bevorzugte nichtionische Polymere.
- Celluloseether, wie Hydroxypropylcellulose, Hydroxyethylcellulose und Methylhydroxypropylcellulose, wie sie beispielsweise unter den Warenzeichen Culminal® und Benecel® (AQUALON) vertrieben werden.
- Schellack
- Polyvinylpyrrolidone, wie sie beispielsweise unter der Bezeichnung Luviskol® (BASF) vertrieben werden.

Beispiele für geeignete anionische Polymere sind:
- Copolymere der Acrylsäure und/oder Methacrylsäure oder deren Ester mit C₁₀₋₃₀-Alkylacrylaten, wie sie beispielsweise unter der Bezeichnung Pemulen® vertrieben werden.
- Polymere und Copolymere der Crotonsäure mit Estern und Amiden der Acryl- und der Methacrylsäure, wie Vinylacetat-Crotonsäure- und Vinylacetat-Vinylpropionat-Crotonsäure-Copolymere. Verbindungen dieser Art sind unter den Markenbezeichnungen Resyn® (NATIONAL STARCH), Luviset® (BASF) und Gafset® (GAF) im Handel; die Produkte Luviset®CA-66 und Luviset®CAP können bevorzugt sein.
- Vinylpyrrolidon/Vinylacrylat-Copolymere, erhältlich beispielsweise unter dem Warenzeichen Luviflex® (BASF). Ein bevorzugtes Polymer ist das unter der Bezeichnung Luviflex® VBM-35 (BASF) erhältliche Vinylpyrrolidon/Acrylat-Terpolymere.
- Acrylsäure/Ethylacrylat/N-tert.Butylacrylamid-Terpolymere, die beispielsweise unter der Bezeichnung Ultrahold® strong (BASF) vertrieben werden, sowie Methacrylsäure/Ethylacrylat/t-Butylacrylat-Terpolymere, die unter der Bezeichnung Luvimer®100P (BASF) vertrieben werden.

Die Verwendung von anionischen, nichtionogenen oder kationischen Polymeren kann in solchen Haarfärbemitteln bevorzugt sein, die frei von Oxidationsfarbstoffvorprodukten sind.
Zur Herstellung der erfindungsgemäßen Färbemittel und Tönungsmittel werden die direktziehenden Farbstoffe und gegebenenfalls die Oxidationsfarbstoffvorprodukte in einen geeigneten wasserhaltigen Träger eingearbeitet. Zum Zwecke der Haarfärbung sind solche Träger z.B. Cremes, Emulsionen, Gele oder auch tensidhaltige schäumende Lösungen, z.B. Shampoos, Schaumaerosole oder andere Zubereitungen, die für die Anwendung auf dem Haar geeignet sind.

Die erfindungsgemäßen Färbemittel und Tönungsmittel können weiterhin alle für solche Zubereitungen bekannten Wirk-, Zusatz- und Hilfsstoffe enthalten. In vielen Fällen enthalten die Mittel mindestens ein Tensid, wobei prinzipiell sowohl anionische als auch zwitterionische, ampholytische, nichtionische und kationische Tenside geeignet sind. In vielen Fällen hat es sich aber als vorteilhaft erwiesen, die Tenside aus anionischen, zwitterionischen oder nichtionischen Tensiden auszuwählen.

Als anionische Tenside eignen sich in erfindungsgemäßen Zubereitungen alle für die Verwendung am menschlichen Körper geeigneten anionischen oberflächenaktiven Stoffe. Diese sind gekennzeichnet durch eine wasserlöslichmachende, anionische Gruppe wie z. B. eine Carboxylat-, Sulfat-, Sulfonat- oder Phosphat-Gruppe und eine lipophile Alkylgruppe mit etwa 10 bis 22 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natri- um-, Kalium- und Ammonium- sowie der Mono-, Di- und Trialkanolammoniumsalze mit 2 oder 3 C-Atomen in der Alkanolgruppe,
- lineare Fettsäuren mit 10 bis 22 C-Atomen (Seifen),
- Ethercarbonsäuren der Formel R-O-(CH₂-CH₂O)ₓ -CH₂-COOH, in der R eine lineare Alkylgruppe mit 10 bis 22 C-Atomen und x = 0 oder 1 bis 16 ist,
- Acylsarcoside mit 10 bis 18 C-Atomen in der Acylgruppe,
- Acyltauride mit 10 bis 18 C-Atomen in der Acylgruppe,
- Acylisethionate mit 10 bis 18 C-Atomen in der Acylgruppe,
- Sulfobernsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen,
- lineare Alkansulfonate mit 12 bis 18 C-Atomen,
- lineare Alpha-Olefinsulfonate mit 12 bis 18 C-Atomen,
- Alpha-Sulfofettsäuremethylester von Fettsäuren mit 12 bis 18 C-Atomen,
- Alkylsulfate und Alkylpolyglykolethersulfate der Formel R-O(CH₂-CH₂O)ₓ-SO₃H, in der R eine bevorzugt lineare Alkylgruppe mit 10 bis 18 C-Atomen und x = 0 oder 1 bis 12 ist,
- Gemische oberflächenaktiver Hydroxysulfonate gemäß DE-A-37 25 030,
- sulfatierte Hydroxyalkylpolyethylen- und/oder Hydroxyalkylenpropylenglykolether gemäß DE-A-37 23 354,
- Sulfonate ungesättigter Fettsäuren mit 12 bis 24 C-Atomen und 1 bis 6 Doppelbindungen gemäß DE-A-39 26 344,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül sowie insbesondere Salze von gesättigten und insbesondere ungesättigten C₈-C₂₂-Carbonsäuren, wie Ölsäure, Stearinsäure, Isostearinsäure und Palmitinsäure.

Als zwitterionische Tenside werden solche oberflächenaktiven Verbindungen bezeichnet, die im Molekül mindestens eine quartäre Ammoniumgruppe und mindestens eine -COO⁽⁻⁾oder -SO₃⁽⁻⁾-Gruppe tragen. Besonders geeignete zwitterionische Tenside sind die sogenannten Betaine wie die N-Alkyl-N,N-dimethylammonium-glycinate, beispielsweise das Kokosalkyl-dimethylammoniumglycinat, N-Acyl-aminopropyl-N,N-dimethylammoniumglycinate, beispielsweise das Kokosacylaminopropyl-dimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Unter ampholytischen Tensiden werden solche oberflächenaktiven Verbindungen verstanden, die außer einer C₈₋₁₈-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C₁₂₋₁₈-Acylsarcosin.

Nichtionische Tenside enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂₋₂₂-Fettsäuremono- und -diester von Anlagerungsprodukten von 1 bis 30 Mol Ethylenoxid an Glycerin,
- C₈₋₂₂-Alkylmono- und -oligoglycoside und deren ethoxylierte Analoga,
- Anlagerungsprodukte von 5 bis 60 Mol Ethylenoxid an Rizinusöl und gehärtetes Rizinusöl,
- Anlagerungeprodukte von Ethylenoxid an Sorbitanfettsäureester,
- Anlagerungsprodukte von Ethylenoxid an Fettsäurealkanolamide.

Beispiele für die in den erfindungsgemäßen Haarbehandlungsmitteln verwendbaren kationischen Tenside sind insbesondere quartäre Ammoniumverbindungen. Bevorzugt sind Ammoniumhalogenide wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid. Weitere erfindungsgemäß verwendbare kationische Tenside stellen die quaternisierten Proteinhydrolysate dar.

Alkylamidoamine, insbesondere Fettsäureamidoamine wie das unter der Bezeichnung Tego Amid®S 18 erhältliche Stearylamidopropyldimethylamin, zeichnen sich neben einer guten konditionierenden Wirkung speziell durch ihre gute biologische Abbaubarkeit aus. Ebenfalls sehr gut biologisch abbaubar sind quaternäre Esterverbindungen, sogenannte "Esterquats", wie die unter dem Warenzeichen Stepantex® vertriebenen Methylhydroxyalkyldialkoyloxyalkyl-ammoniummethosulfate sowie die unter dem Warenzeichen Dehyquart® vertriebenen Produkte wie Dehyquart® AU-46.

Ein Beispiel für ein als kationisches Tensid einsetzbares quaternäres Zuckerderivat stellt das Handelsprodukt Glucquat®100 dar, gemäß INCI-Nomenklatur ein "Lauryl Methyl Gluceth-10 Hydroxypropyl Dimonium Chloride".

Bei den als Tenside eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so daß man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

Weiterhin können die erfindungsgemäßen Haarbehandlungsmittel bevorzugt noch einen konditionierenden Wirkstoff, ausgewählt aus der Gruppe, die von kationischen Tensiden, kationischen Polymeren, Alkylamidoaminen, Paraffinölen, pflanzlichen Ölen und synthetischen Ölen gebildet wird, enthalten.

Kationische Polymere können bevorzugte konditionierende Wirkstoffe sein. In diesem Zusammenhang sei auf die oben genannten Polymere verwiesen.

Als konditionierende Wirkstoffe weiterhin geeignet sind Silikonöle, insbesondere Dialkyl- und Alkylarylsiloxane, wie beispielsweise Dimethylpolysiloxan und Methylphenylpolysiloxan, sowie deren alkoxylierte und quaternierte Analoga. Beispiele für solche Silikone sind die von Dow Corning unter den Bezeichnungen DC 190, DC 200, DC 344, DC 345 und DC 1401 vertriebenen Produkte sowie die Handelsprodukte Q2-7224 (Hersteller: Dow Coming; ein stabilisiertes Trimethylsilylamodimethicon), Dow Corning® 929 Emulsion (enthaltend ein hydroxyl-amino-modifiziertes Silicon, das auch als Amodimethicone bezeichnet wird), SM-2059 (Hersteller: General Electric), SLM-55067 (Hersteller: Wacker) sowie Abil®-Quat 3270 und 3272 (Hersteller: Th. Goldschmidt; diquatemäre Polydimethylsiloxane, Quaternium-80).

Ebenfalls einsetzbar als konditionierende Wirkstoffe sind Paraffinöle, synthetisch hergestellte oligomere Alkene sowie pflanzliche Öle wie Jojobaöl, Sonnenblumenöl, Orangenöl, Mandelöl, Weizenkeimöl und Pfirsichkernöl.

Gleichfalls geeignete haarkonditionierende Verbindungen sind Phospholipide, beispielsweise Sojalecithin, Ei-Lecithin und Kephaline.

Weitere Wirk-, Hilfs- und Zusatzstoffe sind beispielsweise
- Verdickungsmittel wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z.B. Polyvinylalkohol,
- Strukturanten wie Glucose und Maleinsäure,
- Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quatemisierte Proteinhydrolysate,
- Parfümöle, Dimethylisosorbid und Cyclodextrine,
- Lösungsvermittler wie Ethanol, Isopropanol, Ethylenglykol, Propylenglykol, Glycerin und Diethylenglykol,
- Antischuppenwirkstoffe wie Piroctone Olamine und Zink Omadine,
- weitere Substanzen zur Einstellung des pH-Wertes,
- Wirkstoffe wie Panthenol, Pantothensäure, Allantoin, Pyrrolidoncarbonsäuren und deren Salze, Pflanzenextrakte und Vitamine,
- Cholesterin,
- Lichtschutzmittel,
- Konsistenzgeber wie Zuckerester, Polyolester oder Polyolalkylether,
- Fette und Wachse wie Walrat, Bienenwachs, Montanwachs, Paraffine, Fettalkohole und Fettsäureester,
- Fettsäurealkanolamide,
- Komplexbildner wie EDTA, NTA und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,
- Trübungsmittel wie Latex,
- Perlglanzmittel wie Ethylenglykolmono- und -distearat,
- Treibmittel wie Propan-Butan-Gemische, N₂O, Dimethylether, CO₂ und Luft sowie
- Antioxidantien.

Die Bestandteile des wasserhaltigen Trägers werden zur Herstellung der erfindungsgemäßen Färbemittel in für diesen Zweck üblichen Mengen eingesetzt; z.B. werden Emulgiermittel in Konzentrationen von 0,5 bis 30 Gew.-% und Verdickungsmittel in Konzentrationen von 0,1 bis 25 Gew.-% des gesamten Färbemittels eingesetzt.

Es ist prinzipiell möglich, die erfindungsgemäßen Mittel so zu formulieren, daß sie entweder auf dem Haar verbleiben oder wieder aus dem Haar ausgespült werden.

Gemäß einer bevorzugten Ausführungsform werden die erfindungsgemäßen Mittel so formuliert, daß sie auf dem Haar verbleiben können. Dies ist insbesondere bei sogenannten Tönungsmitteln der Fall, aber auch bei Mitteln, die außerdem eine festigende Funktion ausüben sollen.

Die nachfolgenden Beispiele sollen den Erfindungsgegenstand näher erläutern.

### Beispiele

### 1. Herstellung

Eine Mischung aus 23,6g (0,151mol) 4-Fluor-3-nitroanilin, 12,9g (0,151mol) Cyclopentylamin und 12,7g (0,151mol) Natriumhydrogencarbonat wurden in 25ml Wasser und 100ml 2-Propanol für 3h refluxiert. Nach Abkühlen auf Raumtemperatur wurden die Rückstände abfiltriert und das Filtrat mit Chloroform extrahiert. Das Lösemittel wurde im Vakuum entfernt und der Rückstand aus 20%iger Salzsäure umkristallisiert. Es wurde ein goldfarbener Feststoff erhalten, der bei 200°C schmilzt.

### 2. Haarfärbecremes mit direktziehendem Farbstoff

| *Teilmischung A* | |
|---|---|
| Cetearylalkohol | 1,00 g |
| Fettalkoholgemisch auf Basis von Kokosnußöl | 1,00 g |
| Akypo® RLM 45N¹ | 1,10 g |
| p-Hydroxybenzoesäurepropylester | 0,05 g |
| p-Hydroxybenzoesäuremethylester | 0,15 g |
| Wasser | 70,00 g |

| | |
|---|---|
| ¹ Laurylalkohol mit ca. 4,5mol Ethylenoxid-Essigsäure-Natriumsalz (ca.82% Aktivsubstanz; INCI-Bezeichnung: Sodium Laureth-6 Carboxylate) (KAO, Chem-Y) | |

Die Substanzen wurden bei 80°C aufgeschmolzen, mit 80°C heißem Wasser vermischt und unter starkem Rühren emulgiert. Danach wurde die Emulsion unter schwachem Rühren abgekühlt.

| *Teilmischung B* | |
|---|---|
| Ammoniumsulfat | 1,00 g |
| Farbstoff(e) | siehe Tabelle I |
| Ammoniak (25%ige Lösung) | ad pH=9,0 |
| Wasser | 10,00 g |

Die Farbstoffe wurde in 50°C heißem Wasser unter Zugabe von Ammoniumsulfat und Ammoniak gelöst.

Die Farbstofflösung (Teilmischung B) wurde zur Emulsion (Teilmischung A) gegeben, mit Ammoniak auf pH=9 eingestellt und mit Wasser auf 100 g aufgefüllt. Es wurde bis zum Erreichen der Raumtemperatur weitergerührt.

Die so erhaltene Färbecreme wurde auf 5cm lange Strähnen standardisierten, zu 80% ergrauten, aber nicht besonders vorbehandelten Menschenhaares aufgetragen, und dort 30 Minuten bei 32°C belassen. Danach wurde das Haar gespült, mit einem üblichen Haarwaschmittel ausgewaschen und anschließend getrocknet.

Das Ergebnis der Färbeversuche ist der Tabelle I zu entnehmen.

| Direktziehender Farbstoff | Nuance des gefärbten Haares |
|---|---|
| 1,00g 1-(N-cyclopentylamino)-2-nitro-4-aminobenzol | Tiefmagenta |
| 0,10g 1-(N-cyclopentylamino)-2-nitro-4-aminobenzol 0,50g HC Blue No.2 | Parmalila |
| 0,40g 1-(N-cyclopentylamino)-2-nitro-4-aminobenzol 0,25g HC Yellow 4 | Granatbraun |

### 3. Haarfärbecreme mit direktziehendem Farbstoff

| *Teilmischung A* | |
|---|---|
| Hydrenol® D¹ | 6,0 g |
| Lorol® techn.² | 2,0 g |
| Cremophor® A25³ | 2,0 g |
| Wasser | 60,00 g |

| | |
|---|---|
| ¹ C₁₆₋₁₈-Fettalkohol (INCI-Bezeichnung: Cetearyl alcohol) (HENKEL) | |
| ² C₁₂₋₁₈-Fettalkohol (INCI-Bezeichnung: Coconut alcohol) (HENKEL) | |
| ³ Talgalkohol mit ca. 25 EO-Einheiten (INCI-Bezeichnung: Ceteareth-25) (HENKEL) | |

Die Substanzen wurden bei 80°C aufgeschmolzen, mit 80°C heißem Wasser vermischt und unter starkem Rühren emulgiert. Danach wurde die Emulsion unter schwachem Rühren abgekühlt.

| *Teilmischung B* | |
|---|---|
| Ammoniumsulfat | 1,00 g |
| 1-(N-cyclopentylamino)-2-nitro-4-aminobenzol | 0,10g |
| Violet-1,4 D | 0,45g |
| HC Blue 2 | 0,15g |
| HC Red 3 | 0,10g |
| Ammoniak (25%ige Lösung) | ad pH=9,0 |
| Wasser | 10,00 g |

Die Farbstoffe wurde in 50°C heißem Wasser unter Zugabe von Ammoniumsulfat und Ammoniak gelöst.

Die Farbstofflösung (Teilmischung B) wurde zur Emulsion (Teilmischung A) gegeben, mit Ammoniak auf pH=9 eingestellt und mit Wasser auf 100g aufgefüllt. Es wurde bis zum Erreichen der Raumtemperatur weitergerührt.

Die so erhaltene Färbecreme wurde auf 5cm lange Strähnen standardisierten, zu 80% ergrauten, aber nicht besonders vorbehandelten Menschenhaares aufgetragen, und dort 30 Minuten bei 32°C belassen. Danach wurde das Haar gespült, mit einem üblichen Haarwaschmittel ausgewaschen und anschließend getrocknet.
Die Strähne wies eine dunkelviolette Färbung auf.

## Patentansprüche

1. Verwendung von 2-Nitro-p-Phenylendiaminderivaten der Formel (I), 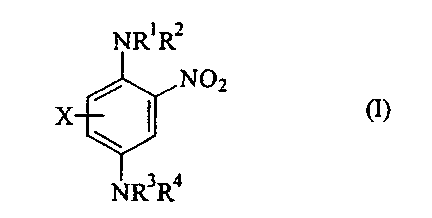 in der R¹ bis R⁴ voneinander unabhängig stehen für Wasserstoff, eine C₁₋₄-Hydroxyalkylgruppe oder einen gesättigten, einfach oder mehrfach ungesättigten C₅-Ring, der gegebenenfalls mit einer C₁₋₄-Alkylgruppe, einem Halogenatom, einer Hydroxygruppe und/oder einer Aminogruppe substituiert sein kann, und X steht für Wasserstoff oder ein Halogenatom, mit der Maßgabe, daß mindestens einer der Substituenten R¹ bis R⁴ ein C₅-Ring ist, als direktziehender Farbstoff zur Verschiebung von Haarfärbungen und -tönungen in den Rotbereich.

2. Verwendung nach Anspruch 1, **dadurch gekennzeichnet, daß** X für Wasserstoff steht.

3. Verwendung nach einem der Ansprüche 1 oder 2, **dadurch gekennzeichnet, daß** R² bis R⁴ für Wasserstoff stehen.

4. Verwendung nach einem der Ansprüche 1 bis 3, **dadurch gekennzeichnet, daß** die Verbindung der Formel (I) 1-(N-cyclopentylamino)-2-nitro-4-aminobenzol ist.

5. Mittel zum Färben und Tönen keratinischer Fasern, insbesondere menschlichen Haaren, **dadurch gekennzeichnet, daß** es als direktziehenden Farbstoff mindestens ein 2-Nitro-p-Phenylendiaminderivat der Formel (I), 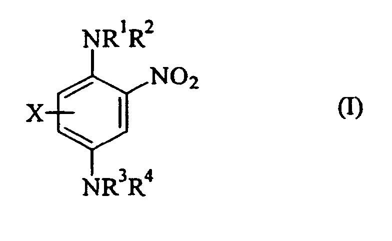 in der R¹ bis R⁴ voneinander unabhängig stehen für Wasserstoff, eine C₁₋₄-Hydroxyalkylgruppe oder einen gesättigten, einfach oder mehrfach ungesättigten C₅-Ring, der gegebenenfalls mit einer C₁₋₄-Alkylgruppe, einem Halogenatom, einer Hydroxygruppe und/oder einer Aminogruppe substituiert sein kann, und X steht für Wasserstoff oder ein Halogenatom, mit der Maßgabe, daß mindestens einer der Substituenten R¹ bis R⁴ ein C₅-Ring ist, enthält.

6. Mittel nach Anspruch 5, **dadurch gekennzeichnet, daß** X für Wasserstoff steht.

7. Mittel nach einem der Ansprüche 5 oder 6, **dadurch gekennzeichnet, daß** R² bis R⁴ für Wasserstoff stehen.

8. Mittel nach einem der Ansprüche 5 bis 7, **dadurch gekennzeichnet, daß** die Verbindung der Formel (I) 1-(N-cyclopentylamino)-2-nitro-4-aminobenzol ist.

9. Mittel nach einem der Ansprüche 5 bis 8, **dadurch gekennzeichnet, daß** es mindestens einen weiteren direktziehenden Farbstoff enthält.

10. Mittel nach einem der Ansprüche 5 bis 9, **dadurch gekennzeichnet, daß** es frei von Oxidationsfarbstoffvorprodukten ist.

11. Mittel nach Anspruch 10, **dadurch gekennzeichnet, daß** es auf dem Haar verbleibt.

12. Mittel nach einem der Ansprüche 10 oder 11, **dadurch gekennzeichnet, daß** es sich um ein haarfestigendes Mittel handelt.

13. Mittel nach einem der Ansprüche 5 bis 9, **dadurch gekennzeichnet, daß** es weiterhin mindestens eine Entwicklerkomponente enthält.

14. Mittel nach Anspruch 13, **dadurch gekennzeichnet, daß** die Entwicklerkomponente ausgewählt ist aus p-Phenylendiamin, p-Toluylendiamin, p-Aminophenol, 1-(2'-Hydroxyethyl)-2,5-diaminobenzol, N,N-Bis-(2-hydroxy-ethyl)-p-phenylendiamin, 4-Amino-3-methylphenol, 4-Amino-2-((diethylamino)-methyl)-phenol, 2-Aminomethyl-4-aminophenol, 2,4,5,6-Tetraaminopyrimidin, 2-Hydroxy-4,5,6-triaminopyrimidin, 4-Hydroxy-2,5,6-triaminopyrimidin und 4,5-Diamino-1-(2'-hydroxyethyl)-pyrazol.

15. Mittel nach einem der Ansprüche 13 oder 14, **dadurch gekennzeichnet, daß** es weiterhin eine Kupplerkomponente, ausgewählt aus der Gruppe, die von 1-Naphthol, 1,5-, 2,7- und 1,7-Dihydroxynaphthalin, 3-Aminophenol, 5-Amino-2-methylphenol. Resorcin, 4-Chlorresorcin, 2-Chlor-6-methyl-3-aminophenol, 2-Methylresorcin, 5-Methylresorcin, 2,5-Dimethylresorcin und 2,6-Dihydroxy-3,4-diaminopyridin gebildet wird, enthält.

16. Mittel nach einem der Ansprüche 5 bis 15, **dadurch gekennzeichnet, daß** es weiterhin ein anionisches, nichtionogenes oder kationisches Polymer enthält.

17. Mittel nach einem der Ansprüche 5 bis 16, **dadurch gekennzeichnet, daß** es mindestens ein Tensid enthält.

18. Mittel nach einem der Ansprüche 5 bis 17, **dadurch gekennzeichnet, daß** es mindestens eine konditionierende Komponente enthält.

## Claims

1. The use of 2-nitro-p-phenylenediamine derivatives corresponding to formula (I): 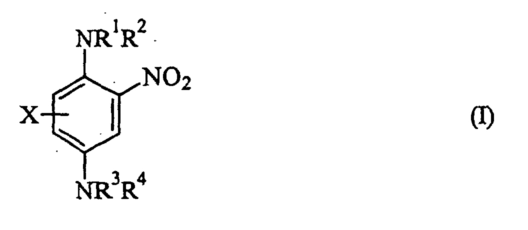 in which R¹ to R⁴ independently of one another represent hydrogen, a C₁₋₄ hydroxyalkyl group or a saturated, mono- or polyunsaturated C₅ ring which may optionally be substituted by a C₁₋₄ alkyl group, a halogen atom, a hydroxy group and/or an amino group and X is hydrogen or a halogen atom, with the proviso that at least one of the substituents R¹ to R⁴ is a C₅ ring,
as a substantive dye for shifting hair colors and tints into the red region.

2. The use claimed in claim 1, **characterized in that** X stands for hydrogen.

3. The use claimed in claim 1 or 2, **characterized in that** R² to R⁴ stand for hydrogen.

4. The use claimed in any of claims 1 to 3, **characterized in that** the compound corresponding to formula (I) is 1-(N-cyclopentylamino)-2-nitro-4-aminobenzene.

5. A preparation for coloring and tinting keratin fibers, more particularly human hair, **characterized in that** it contains as substantive dye at least one 2-nitro-p-phenylenediamine derivative corresponding to formula (I): 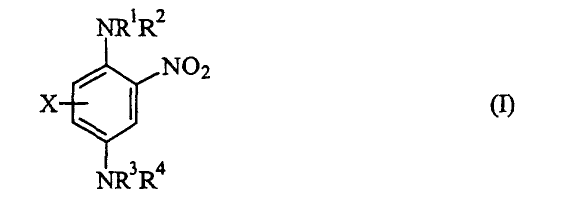 in which R¹ to R⁴ independently of one another represent hydrogen, a C₁₋₄ hydroxyalkyl group or a saturated, mono- or polyunsaturated C₅ ring which may optionally be substituted by a C₁₋₄ alkyl group, a halogen atom, a hydroxy group and/or an amino group and X is hydrogen or a halogen atom, with the proviso that at least one of the substituents R¹ to R⁴ is a C₅ ring.

6. A preparation as claimed in claim 5, **characterized in that** X stands for hydrogen.

7. A preparation as claimed in claim 5 or 6, **characterized in that** R² to R⁴ stand for hydrogen.

8. A preparation as claimed in any of claims 5 to 7, **characterized in that** the compound corresponding to formula (I) is 1-(N-cyclopentylamino)-2-nitro-4-aminobenzene.

9. A preparation as claimed in any of claims 5 to 8, **characterized in that** it additionally contains at least one other substantive dye.

10. A preparation as claimed in any of claims 5 to 9, **characterized in that** it is free from oxidation dye precursors.

11. A preparation as claimed in claim 10, **characterized in that** it remains on the hair.

12. A preparation as claimed in claim 10 or 11, **characterized in that** it is a hair-setting preparation.

13. A preparation as claimed in any of claims 5 to 9, **characterized in that** it additionally contains at least one primary intermediate.

14. A preparation as claimed in claim 13, **characterized in that** the primary intermediate is selected from p-phenylenediamine, p-toluylenediamine, p-aminophenol, 1-(2'-hydroxyethyl)-2,5-diaminobenzene, N,N-bis-(2-hydroxyethyl)-p-phenylenediamine, 4-amino-3-methylphenol, 4-amino-2-((diethylamino)-methyl)-phenol, 2-aminomethyl-4-aminophenol, 2,4,5,6-tetraaminopyrimidine, 2-hydroxy-4,5,6-triaminopyrimidine, 4-hydroxy-2,5,6-triaminopyrimidine and 4,5-diamino-1-(2'-hydroxyethyl)-pyrazole.

15. A preparation as claimed in claim 13 or 14, **characterized in that** it additionally contains a secondary intermediate selected from the group consisting of 1-naphthol, 1,5-, 2,7- and 1,7-dihydroxynaphthalene, 3-aminophenol, 5-amino-2-methylphenol, resorcinol, 4-chlororesorcinol, 2-chloro-6-methyl-3-aminophenol, 2-methyl resorcinol, 5-methyl resorcinol, 2,5-dimethyl resorcinol and 2,6-dihydroxy-3,4-diaminopyridine.

16. A preparation as claimed in any of claims 5 to 15, **characterized in that** it additionally contains an anionic, nonionic or cationic polymer.

17. A preparation as claimed in any of claims 5 to 16, **characterized in that** it contains at least one surfactant.

18. A preparation as claimed in any of claims 5 to 17, **characterized in that** it contains at least one conditioning component.

## Revendications

1. Utilisation de dérivés de 2-nitro-p-phénylènediamine répondant à la formule (I) 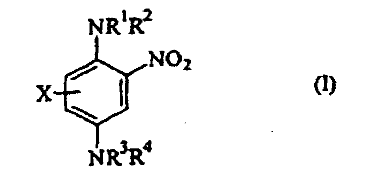 dans laquelle R¹ à R⁴ représentent, indépendamment l'un de l'autre, un atome d'hydrogène, un groupe hydroxyalkyle en C₁-C₄ ou bien un noyau en C₅ saturé, une fois ou plusieurs fois insaturé, qui peut être substitué, le cas échéant, avec un groupe alkyle en C₁-C₄, avec un atome d'halogène, avec un groupe hydroxyle et/ou avec un groupe amino, et X représente un atome d'hydrogène ou un atome d'halogène, à condition qu'au moins un des substituants R¹ à R⁴ représente un noyau en C₅, à titre de colorant montant directement sur la fibre pour le déplacement de colorations et de teintes de cheveux dans la gamme des rouges.

2. Utilisation selon la revendication 1, **caractérisée en ce que** X représente un atome d'hydrogène.

3. Utilisation selon l'une quelconque des revendications 1 ou 2, **caractérisée en ce que** R² à R⁴ représentent un atome d'hydrogène.

4. Utilisation selon l'une quelconque des revendications 1 à 3, **caractérisé en ce que** le composé répondant à la formule (I) est le 1-(N-cyclopentylamino)-2-nitro-4-aminobenzène.

5. Agent pour la coloration et la teinture de fibres kératiniques, en particulier de cheveux humains, **caractérisé en ce qu'**il contient, à titre de colorant montant directement sur la fibre, au moins un dérivé de 2-nitro-p-phénylènediamine répondant à la formule (I) 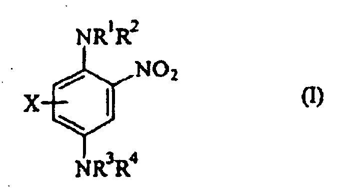 dans laquelle R¹ à R⁴ représentent, indépendamment l'un de l'autre, un atome d'hydrogène, un groupe hydroxyalkyle en C₁-C₄ ou bien un noyau en C₅ saturé, une fois ou plusieurs fois insaturé, qui peut être substitué, le cas échéant, avec un groupe alkyle en C₁-C₄, avec un atome d'halogène, avec un groupe hydroxyle et/ou avec un groupe amino, et X représente un atome d'hydrogène ou un atome d'halogène, à condition qu'au moins un des substituants R¹ à R⁴ représente un noyau en C₅.

6. Agent selon la revendication 5, **caractérisée en ce que** X représente un atome d'hydrogène.

7. Agent selon l'une quelconque des revendications 5 ou 6, **caractérisée en ce que** R² à R⁴ représentent un atome d'hydrogène.

8. Agent selon l'une quelconque des revendications 5 à 7, **caractérisé en ce que** le composé répondant à la formule (I) est le 1-(N-cyclopentylamino)-2-nitro-4-aminobenzène.

9. Agent selon l'une quelconque des revendications 5 à 8, **caractérisé en ce qu'**il contient au moins un autre colorant montant directement sur la fibre.

10. Agent selon l'une quelconque des revendications 5 à 9, **caractérisé en ce qu'**il est exempt de précurseurs de colorants d'oxydation.

11. Agent selon la revendication 10, **caractérisé en ce qu'**il reste sur les cheveux.

12. Agent selon l'une quelconque des revendications 10 ou 11, **caractérisé en ce qu'**il s'agit d'un agent renforçant les cheveux.

13. Agent selon l'une quelconque des revendications 5 à 9, **caractérisé en ce qu'**il contient en outre au moins un composant développeur

14. Agent selon la revendication 13, **caractérisé en ce que** le composant développeur est choisi parmi le groupe comprenant la p-phénylènediamine, la p-toluylènediamine, le p-aminophénol, le 1-(2'-hydroxyéthyl)-2,5-diaminobenzène, la N,N-bis-(2-hydroxyéthyl)-p-phénylènediamine, le 4-amino-3-méthylphénol, le 4-amino-2-((diéthylamino)-méthyl)-phénol, le 2-aminométhyl-4-aminophénol, la 2,4,5,6-tétraaminopyrimidine, la 2-hydroxy-4,5,6-triamino-pyrimidine, la 4-hydroxy-2,5,6-triaminopyrimidine et le 4,5-diamino-1-(2'-hydroxyéthyl)-pyrazole.

15. Agent selon l'une quelconque des revendications 13 ou 14, **caractérisé en ce qu'**il contient en outre un composant de couplage, choisi parmi le groupe qui est formé par le 1-naphtol, le 1,5-, le 2,7- et le 1,7-dihydroxynaphtalène, le 3-aminophénol, le 5-amino-2-méthyl phénol, le résorcinol, le 4 chlororésorcinol, le 2-chloro-6-méthyl-3-amino-phénol, le 2-méthylrésorcinol, le 5-méthylrésorcinol, le 2,5-diméthyl-résorcinol et la 2,6-dihydroxy-3,4-diaminopyridine.

16. Agent selon l'une quelconque des revendications 5 à 15, **caractérisé en ce qu'**il contient en outre un polymère anionique, non ionogène ou cationique.

17. Agent selon l'une quelconque des revendications 5 à 16, **caractérisé en ce qu'**il contient au moins un agent tensioactif.

18. Agent selon l'une quelconque des revendications 5 à 17, **caractérisé en ce qu'**il contient au moins un composant de conditionnement.
